(19) Europäisches Patentamt
European Patent Office
Office européen des brevets

(11) Veröffentlichungsnummer: **0 213 330 A2**

# (12) EUROPÄISCHE PATENTANMELDUNG

(21) Anmeldenummer: **86109412.6**

(22) Anmeldetag: **10.07.86**

(51) Int. Cl.4: **C07D 493/10**, C07D 301/14, //(C07D493/10,319:00,303:00)

(30) Priorität: **05.08.85 DE 3528003**

(43) Veröffentlichungstag der Anmeldung: **11.03.87 Patentblatt 87/11**

(84) Benannte Vertragsstaaten: **AT CH DE FR GB IT LI NL SE**

(71) Anmelder: **Degussa Aktiengesellschaft**
**Weissfrauenstrasse 9**
**D-6000 Frankfurt am Main 1(DE)**

(72) Erfinder: **Boehme, Georg**
**Nordring 49**
**D-6458 Rodenbach 1(DE)**
Erfinder: **Hofen, Willi**
**Südring 54**
**D-6458 Rodenbach 1(DE)**
Erfinder: **Grund, Andreas, Dr.**
**Rilkeweg 15**
**D-6100 Darmstadt(DE)**
Erfinder: **Petsch, Heinrich**
**Tulpenstrasse 23**
**D-6450 Hanau 8(DE)**
Erfinder: **Prescher, Günter, Dr.**
**Liesingstrasse 2**
**D-6450 Hanau 9(DE)**

## (54) Verfahren zur Herstellung eines cycloaliphatischen Diepoxids.

(57) In technisch einfacher Form läßt sich das Diepoxid der Formel

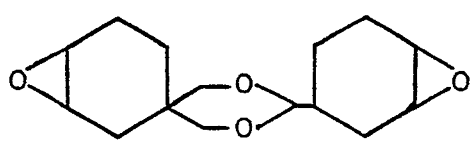

mit benzolischer Perpropionsäure, die auch in ungereinigter Form mit bestimmten Maximalgehalten an Wasserstoffperoxid, Wasser und Mineralsäure verwendet werden kann, aus dem entsprechenden Diolefin herstellen.

EP 0 213 330 A2

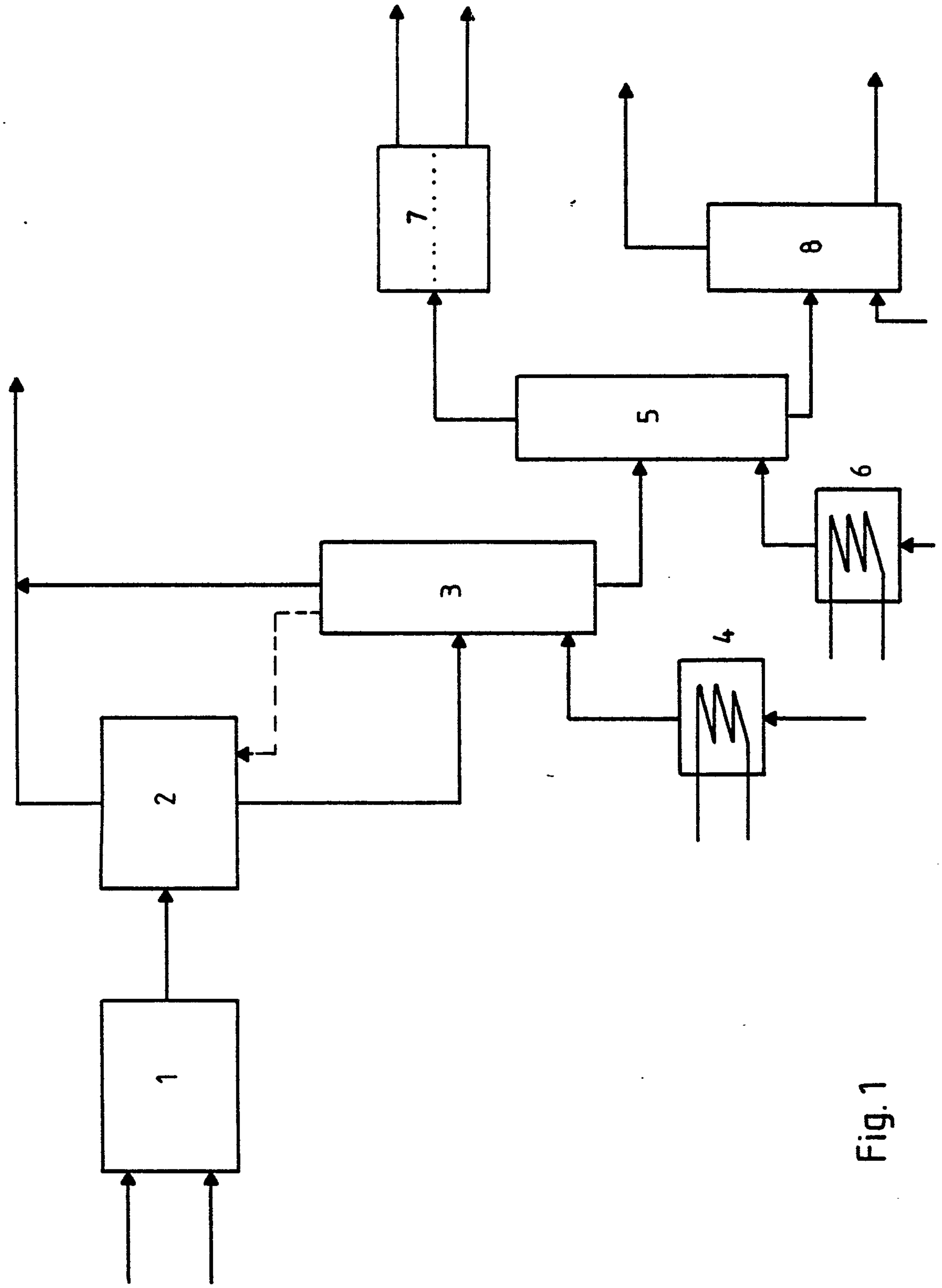


Fig. 1

# Verfahren zur Herstellung eines cycloaliphatischen Diepoxids

Die vorliegende Erfindung betrifft ein Verfahren zur Herstellung eines cycloaliphatischen Diepoxids der Formel

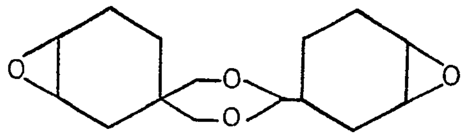

durch Epoxidation eines Diolefins der Formel

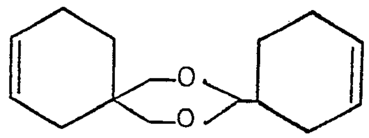

mit einer Percarbonsäure in organischem Lösungsmittel sowie zur Aufarbeitung des dabei entstehenden Reaktionsgemisches.

Das cycloaliphatische Diepoxid obenstehender Struktur findet zunehmend Verwendung als Bestandteil von Epoxidharzen, die sich vor allem als Isolations-und Vergußmaterial auf dem Elektro-und Elektroniksektor sowie für strahlungshärt bare Lack-und Beschichtungssysteme eignen. Gerade letztere sind besonders umweltfreundlich, da sie frei von Lösungsmitteln sind.

Es ist seit langem bekannt, Epoxide durch Umsetzung von Olefinen mit Chlor im alkalischen Medium und nachfolgender Behandlung mit Basen herzustellen (Ullmann's Enzyklopädie der technischen Chemie, 3. Auflage, Band 10, Seite 565). Der wesentliche Nachteil dieses Verfahrens sind die erheblichen Mengen umweltbelastender Abwässer, die bei diesem Prozeß zwangsweise anfallen.

Es ist ferner bekannt, daß Ethylen mit guten Ausbeuten in der Gasphase mit molekularem Sauerstoff an einem silberhaltigen Katalysator epoxidiert werden kann. Wegen seiner mangelnden Selektivität ist jedoch dieses Verfahren für andere Olefine ungeeignet.

Weiterhin lassen sich Olefine durch Umsetzung mit Hydroperoxiden, die aus Kohlenwasserstoffen, wie z.B. Isobutan oder Ethylbenzol, durch Oxidation mit Luft erhältlich sind (US-PS 3 351 635), in Gegenwart eines Katalysators, der Vanadin-, Molybdän-oder Wolframverbindungen enthält, in entsprechende Epoxide überführen. Diese Methode hat den gewichtigen Nachteil, daß neben der erforderlichen Abtrennung des Katalysatorsystems der aus dem Hydroperoxid als Koppelprodukt in äquimolarem Maßstab anfallende Alkohol, sofern er nicht wirtschaftlich verwertbar ist, nur unter erheblichem technischen Aufwand in das Hydroperoxid rückgeführt werden kann.

Durch Anwendung der "Prileschajew-Reaktion" (N. Prileschajew, Ber. dtsch. chem. Ges. 42 , 4811 (1909)) können die zuvor erwähnten Nachteile teilweise vermieden bzw. vermindert werden.

Diese Reaktion beinhaltet im wesentlichen die Umsetzung eines Olefins mit einer organischen Percarbonsäure. Allerdings führt auch hierbei z.B. die Verwendung von Perameisensäure, die darüberhinaus in höheren Konzentrationen detonationsfähige Gemische ausbildet, zu erheblichen Abwassermengen, die einer gewissenhaften Entsorgung bedürfen. Auch ein Einsatz von Peressigsäure in wäßrigem Medium führt zu großen Mengen verdünnter Essigsäure, die auf wirtschaftliche Weise nicht aufkonzentriert und rückgeführt werden können. Falls die Peressigsäure, wie häufig notwendig wegen der Produktstabilität, während des Prozesses mit Alkalicarbonatlösung abgepuffert und/oder nach der Reaktion mit Alkalihydroxidlösung neutralisiert wird, fallen stark salzhaltige, die Umwelt belastende Abwässer an.

Es ist bekannt, das vorgenannte cycloaliphatische Diepoxid durch Epoxidation des entsprechenden Diolefins in Ethylacetat mit wäßriger Peressigsäure unter gleichzeitiger Abpufferung mit Natriumcarbonatlösung herzustellen (Ullmann's Enzyklopädie der technischen Chemie, 3. Auflage, Band 10, Seite 574). Nach der Reaktion wird mit Natronlauge neutralisiert und mehrere Male mit Natronlauge und Natriumsulfatlösung nachgewaschen. Sehr nachteilig ist nicht allein die lange Reaktionsdauer von mehr als 6 Stunden, sondern auch die großen Abwassermengen mit erheblicher Salzfracht, ferner geht die Peressigsäure als Natriumacetat verloren.

Nach GB-PS 922 206 kann das erfindungsgemäß beanspruchte Diepoxid durch Epoxidation des entsprechenden Olefins mit in situ erzeugter Perbernsteinsäure erfolgen. Nach diesem Verfahren beträgt die Ausbeute lediglich 86 %, eingesetztes Bernsteinsäureanhydrid geht mit dem Abwasser bei der Aufarbeitung verloren, so daß eine technische Durchführung nicht wirtschaftlich ist. In der gleichen Patentschrift wird mit dem Oxidationsagens Peroxoiminoessigsäure, die aus Aceto nitril und Wasserstoffperoxid hergestellt wird, in Gegenwart von Natriumcarbonat epoxidiert. Auch dieses Verfahren ist im Sinne einer technischen Anwendung nicht vorteilhaft, da das Acetonitril als Acetamid im Abwasser verlorengeht.

Im allgemeinen vertritt die Fachwelt über Epoxidation mit Persäuren die Meinung, daß die derartig mit Persäuren erhaltenen Reaktionsgemische aufgrund ihres Gehaltes an Wasser und Säure, z.B. Essigsäure, sehr leicht mit den gebildeten Epoxiden unter Bildung von Nebenprodukten, wie Glykolen, Glykolmono-und diestern reagieren, siehe DE-AS 15 43 032. Epoxidationsverfahren, die z.B. Perameisen-bis Perpropionsäure verwendeten, galten daher als besonders schwierig durchführbar in saurem Milieu, da hierdurch Aufspaltung des Oxiranringes eintrat, siehe DE-PS 29 16 834.

In diesem Zusammenhang ist es auch zu verstehen, daß in der DE-OS 31 01 037 und EP-OS 056 932, die die Herstellung von n-Alkyloxiranen mittels Perpropionsäure beschreiben, im Anspruch 1 eine Percarbonsäurelösung mit einem Mineralsäuregehalt kleiner als 50 ppm beansprucht wird. Laut Beschreibung im Text soll der Mineralsäuregehalt bevorzugt sogar kleiner als 10 ppm betragen. Diese Verfahren bezogen sich auf die Herstellung von Monoepoxiden.

Nach dem genannten Stand der Technik war aber zu erwarten, daß sich Diepoxide noch - schlechter mit Percarbonsäuren herstellen lassen würden, da das Molekül wegen zwei vorhandener Epoxidteilstrukturen besonders leicht zu Nebenreaktionen neigt.

Aufgabe der Erfindung ist es, das vorgenannte Diolefin mit Hilfe von Perpropionsäure in das entsprechende Diepoxid umzuwandeln, und zwar in guten Ausbeuten unter Vermeiden von störender Nebenproduktbildung.

Es wurde nun gefunden, daß sich diese Aufgabe lösen läßt, wenn man das Diolefin der Formel

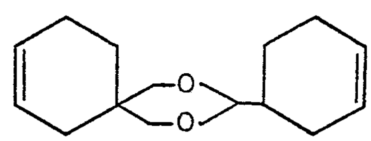

mit einer benzolischen Lösung von Perpropionsäure im Molverhältnis 1 : 2 bis 1 : 3 (Diolefin zu Perpropionsäure) bei einer Temperatur von 10 bis 100 °C umsetzt.

Perpropionsäure kann z.B. gemäß dem in der DE-PS 25 19 289 beschriebenen Verfahren hergestellt werden, indem man wäßriges Wasserstoffperoxid mit Propionsäure in Gegenwart von Schwefelsäure umsetzt und anschließend die entstandene Perpropionsäure mit Benzol aus dem Reaktionsgemisch extrahiert. Die auf diese Weise erhaltene Perpropionsäure in benzolischer Lösung kann noch weiter gereinigt werden, um den Restgehalt an Schwefelsäure, Wasser und Wasserstoffperoxid zu verringern, siehe z.B. DE-PS 25 19 290.

Bevorzugt ist aber eine Perpropionsäurelösung, die keiner weiteren Reinigung bedarf, vielmehr läßt sich der Rohextrakt aus der Persäureherstellung ohne Nachteile einsetzen, was zu einem erheblich verringerten technischen Aufwand führt und daher das Verfahren sehr wirtschaftlich gestaltet.

Es kann daher eine Perpropionsäurelosung in Benzol verwendet werden, die bis zu 1,5 Gewichtsprozent Wasserstoffperoxid, 1,5 Gewichtsprozent Wasser und bis zu 800 ppm Mineralsäure enthält.

Nach dem erfindungsgemäßen Verfahren wird als Ausgangskomponent 2-(Cyclo-3-hexenyl)-spiro-(1,3-dioxan-5,3'-cyclohexen) eingesetzt. Dieses ist z.B. durch Acetalisierung von 3-Cyclohexen-1-carboxaldehyd mit 1,1-Bis-hydroxymethyl-3-cyclohexen zugänglich.

Nach dem erfindungsgemäßen Verfahren wird das Diolefin als solches, oder auch verdünnt bzw. gelöst in einem geeigneten Lösungsmittel, vorzugsweise Benzol, eingesetzt werden, wobei man die Konzentration in einem weiten Bereich frei wählt.

Die Lösungen der Perpropionsäure, die überwiegend aus Benzol, Propionsäure und Perpropionsäure bestehen, können zwischen 10 und 30 Gewichtsprozent der Persäure enthalten. Vorzugsweise werden Lösungen mit einem Persäuregehalt von ca. 20 Gewichtsprozent eingesetzt.

Ein bevorzugtes Molverhältnis von eingesetztem Diolefin zu Perpropionsäure liegt bei 1 : 2 bis 1 : 2,4. Besonders bevorzugt ist ein Persäureüberschuß von 3 bis 15 Molprozent.

Die erfindungsgemäße Umsetzung findet vorzugsweise bei 20 bis 50 °C statt. Das erfindungsgemäße Verfahren läßt sich unter verschiedenen Drücken durchführen; im allgemeinen wird unter Normaldruck gearbeitet, das Verfahren läßt sich aber auch bei Über-oder Unterdruck durchführen.

Die Umsetzung kann sowohl diskontinuierlich oder kontinuierlich in für diese Art der Reaktion geeigneten Reaktoren, wie Rührkesseln, Rührkesselkaskaden, Röhren-oder Schlaufenreaktoren erfolgen, wobei die Reaktionswärme auf beliebige Weise, z.B. Siedekühlung oder innen-bzw. außenliegende Kühleinrichtungen abgeführt wird.

Geeignete Werkstoffe für die Reaktionsapparate zur Durchführung des erfindungsgemäßen Verfahrens sind z.B. Glas, Edelstahl oder emailliertes Material.

Die Perpropionsäure wird mit dem Diolefin bzw. dessen Lösung in einem geeigneten Lösungsmittel, auf beliebige Art zusammengebracht. So kann man beide Reaktionsteilnehmer zu sammen oder nacheinander in beliebiger Reihenfolge in den Reaktor einbringen. Bei diskontinuierlicher Arbeitsweise wird vorzugsweise das Diolefin vorgelegt und die Persäure unter Kontrolle der Reaktionstemperatur zudosiert. Es kann aber ebenso umgekehrt verfahren werden, d.h., man legt die Persäure vor und dosiert das Olefin unter Kontrolle der Reaktionstemperatur zu. Erfolgt die Umsetzung kontinuierlich, so können beide Reaktanten getrennt oder gemeinsam dem Reaktor zugeführt werden. Bei Verwendung mehrerer hintereinandergeschalteter Reaktoren, wie z.B. einer Rührkesselkaskade oder einer Folge von Rührkesseln mit einem Rohrreaktor als Nachreaktor kann man sowohl Persäure-als auch Diolefinzugabe auf mehrere Reaktoren verteilen. Geeignete Lösungsmittel sind neben Benzol Toluol, Chlorbenzol, halogenierte Kohlenwasserstoffe, wie Methylenchlorid, Chloroform, Tetrachlorkohlenstoff für Diolefin, besonders bevorzugt wird Benzol.

Nach dem erfindungsgemäßen Verfahren ist eine kontinuierliche Arbeitsweise besonders vorteilhaft. Gemäß dieser wird das cycloaliphatische Diolefin bzw. seine Lösung mit einer Lösung von Perpropionsäure in Benzol im Molverhältnis 1 : 2 bis 1 : 3 bei den genannten Temperaturen von 10 bis 100 °C (bevorzugt 20 bis 50 °C) in ein Reaktionssystem eingespeist, das aus einer Folge von 1 bis 4 ideal durchmischten Reaktoren und einem Nachreaktor besteht, wobei man die Verweilzeit so einstellt, daß der Umsatz, bezogen auf eingesetzte olefinische Doppelbindungen nach dem oder den ideal durchmischten Reaktor(en) mindestens 80 Molprozent und nach dem Nachreaktor mindestens 95, bevorzugt>98 Molprozent, beträgt. Anschließend wird das den Nachreaktor verlassende Reaktionsgemisch in einer Kombination von Destillations-und Desorptionsschritten von Benzol, Propionsäure, unumgesetzter Perpropionsäure sowie sonstigen flüchtigen Bestandteilen befreit. Diese Auftrennung des Reaktionsgemisches kann, da das gebildete Diepoxid die Komponente mit dem höchsten Siedepunkt des Gemisches darstellt, nach einer der folgenden Varianten durchgeführt werden.

Variante 1 (diskontinuierlich)

Gemäß dieser werden die einzelnen Bestandteile des Reaktionsgemisches in der Reihenfolge ihrer Siedepunkte einzeln oder als Gemische destillativ oder destillativ und desorptiv entfernt. Hierbei gehen Fraktionen von Benzol, Resten Perpropionsäure, Propionsäure und sonstige leicht flüchtige Bestandteile über. Als Sumpf verbleibt das Diepoxid. Das abgetrennte Benzol sowie die Propionsäure können gegebenenfalls nach weiteren Reinigungsschritten in die Persäureherstellung rückgeführt werden.

Variante 2 (kontinuierlich, Abbildung 1)

Nach dieser kontinuierlich durchzuführenden Variante werden zunächst, nachdem das Reaktionsgemisch die Reaktionseinheit 1 verlassen hat, Benzol, Propionsäure und nichtumgesetzte Perpropionsäure größtenteils in der ein-oder mehrstufigen Destillationseinheit 2 entfernt. Diese besteht aus geeigneten Apparaten, wie Dünnschicht-, Fallfilm-oder Umlaufverdampfern. Es ist vorteilhaft unter vermindertem Druck von 0,5 bis 600, vorzugsweise 10 bis 300 mbar, zu destillieren - (Temperatur des Heizmediums 50 bis 150 °C). Die mittleren Verweilzeiten, bezogen auf die einzelnen Stufen der Verdampfung liegen bei maximal 10 Minuten, bevorzugt werden Verweilzeiten von maximal 5 Minuten. Anschließend wird nach dem erfindungsgemäßen Verfahren die im Rohprodukt verbliebene Menge Propionsäure in der Desorptionseinheit 3 mit Benzoldampf, der im Verdampfer 4 generiert wird, desorptiv entfernt. Die Brüden aus der Desorptionseinheit 3 können entweder an der Destillationseinheit 2 vorbeigeführt oder durch diese hindurchgeführt werden. Nach diesem Schritt werden aus dem Diepoxid die verbliebenen Spuren Benzol mit Wasserdampf aus dem Ver dampfer 6 in der Desorptionseinheit 5 und/oder Stickstoff bzw. sonstigen Inertgasen in der Desorptionseinheit 8 desorbiert. Es ist besonders bevorzugt, zunächst mit Wasserdampf und anschließend mit Inertgasen zu desorbieren. Das aus der Desorptionseinheit 5 stammende Kondensat trennt sich im Phasentrenner 7 in eine organische Phase und Wasser, welches dem Verdampfer 6, gegebenenfalls nach Ergänzung, zurückgeführt werden kann. Die organische Phase, die vorwiegend Benzol und Propionsäure enthält, wird gegebenenfalls nach weiterer Aufarbeitung der Perpropionsäureherstellung oder Epoxidation zugeführt. Ebenfalls werden die aus den Destillations-bzw. Desorptionseinheiten 2 und 3 stammenden Kondensatströme, die im wesentlichen aus Benzol, nichtumgesetzter Perpropion-und Propionsäure bestehen, nach weiterer Auftrennung -siehe Abbildung 3 -, deren Beschreibung später erfolgt, in die Persäureherstellung bzw. Epoxidation rückgeführt.

Variante 3 (kontinuierlich, Abbildung 2)

Gemäß der kontinuierlich anzuwendenden Variante 3 werden, wie bei Variante 2, Benzol, unumgesetzte Perpropionsäure und Propionsäure in der ein-oder mehrstufigen Destillationseinheit 2 entfernt. Anschließend wird in der Desorptionseinheit 3 restliche Propionsäure mit Benzoldampf desorbiert. Zur Entfernung von verbliebenen Spuren an Propionsäure wird nun das Rohepoxid, welches zur Verringerung der Viskosität mit Benzol verdünnt werden kann, mit wäßrigen Alkalien in der Extraktion 9 und anschließend mit Wasser in der ein-oder mehrstufigen Extraktionseinheit 10 gewaschen. Geeignete Apparate für diese Schritte sind Extraktionskolonnen verschiedener Bauart oder auch Mixer-Settler-Einheiten, deren Betriebsweise und Auslegung dem Fachmann wohlbekannt sind. Als wäßrige Alkalilösungen können Lösungen, wie z.B. NaOH, KOH, $Na_2CO_3$, $K_2CO_3$, $NaHCO_3$, $KHCO_3$, $NH_3$ usw. eingesetzt werden, wobei deren Konzentration in einem wei ten Bereich frei wählbar ist. Bevorzugt wird eine NaOH-Lösung mit einer Konzentration zwischen 0,01 und 10 Gewichtsprozent, besonders zwischen 0,1 und 2,0 Gewichtsprozent. Bei Einsatz von Mixer-Settler-Einheiten für die Wäsche mit Wasser kann das Wasser im Gegenstrom geführt werden, es kann aber auch jede Einheit mit Frischwasser betrieben werden. Vorteilhaft wird ein Teil des Abwassers aus den Mixer-Settler-Einheiten zum Ansetzen der Alkali-Lösung verwendet. Die Alkali-und Wasserwäsche kann in einem Temperaturbereich von 10 bis 90 °C betrieben werden, bevorzugt werden Temperaturen von 30 bis 70 °C. In der Alkali-Wäsche beträgt das Gewichtsverhältnis zwischen durchgesetztem Epoxid zu Alkalilösung 1 : 1 bis 1 : 100, in der Wasserwäsche liegt das Verhältnis von Epoxiddurchsatz zu Wasserdurchsatz bei 1 : 1 bis 1 : 100.

Im Anschluß an die Wasserwäsche erfolgt die weitere Aufarbeitung durch Desorption mit Wasserdampf und/oder Inertgas wie bei Variante 2 beschrieben.

Die bei allen Varianten durch Kombination von Destillations-und Desorptionsschritten anfallenden Kondensate, die überwiegend aus Benzol, unumgesetzter Perpropionsäure, Propionsäure und sonstigen Leichtsiedern bestehen, werden nach dem erfindungsgemäßen Verfahren in eine aus einer oder mehreren Kolonnen bestehende Destillationseinheit 11 (Abbildung 3) überführt. Diese liefert als Kopfprodukt Benzol und gegebenenfalls weitere Leichtsieder. Ersteres wird gegebenenfalls nach weiterer Reinigung in 12 in das Herstellungsverfahren der Perpropionsäure zurückgeführt. Im Sumpf der Destillationseinheit 11 fällt ein Gemisch aus Propionsäure, Perpropionsäure und Benzol mit einem Benzolanteil von 5 bis 35 Gewichtsprozent, bezogen auf die Sumpfmischung, an. Dieses Gemisch wird einer weiteren Destillationseinheit 13 zugeführt, in der man die Gesamtmenge des zugeführten Benzols und der Perpropionsäure mit Anteilen Propionsäure über Kopf abzieht und dabei eine Konzentration von Perpropionsäure im Destillat von 25 Gewichtsprozent nicht überschreitet und dieses Kopfprodukt in das Herstellungsverfahren der Perpropionsäure oder in die Umsetzung des Diolefins mit Perpropionsäure zurückführt. Als Sumpfprodukt in Kolonne 13 fällt Propionsäure an, die nach weiterer Aufarbeitung wie Reindestillation in die Herstellung der Perpropionsäure gegebenenfalls nach Ergänzung zurückgeführt wird. Besonders vorteilhaft ist es, die in 13 anfallende Propionsäure dampfförmig oberhalb des Sumpfes abzuziehen und zu kondensieren, da hierdurch der weitere Reinigungsschritt entfällt.

Erfindungsgemäß werden alle destillativen Aufarbeitungsschritte vorzugsweise unter vermindertem Druck, z.B. 0,5 bis 600 mbar, durchgeführt. Kolonnen, in denen Benzol oder Propionsäure als Kopfprodukt anfällt, können ebenso bei Normaldruck betrieben werden.

Das erfindungsgemäße Verfahren bietet eine Reihe von überraschenden Vorteilen.

Mit Hilfe der sogenannten Prileschajew-Reaktion ist es nach diesem Verfahren möglich, das sogenannte Diepoxid im technischen Maßstab auf gefahrlose Weise in hoher Ausbeute herzustellen. Das auf diese Weise erhaltene Produkt zeichnet sich durch außerordentliche Reinheit, hohen Epoxidgehalt, Geruchlosigkeit und helle Farbe auf. Ebenfalls besonders niedrig ist der Gehalt an Monoepoxid sowie ionischen Verunreinigungen, wodurch ein Produkt mit deutlich besseren Eigenschaften als nach anderen Verfahren hergestelltes Diepoxid vorstehender Struktur zur Verfügung steht. Gerade für Anwendungen im Bereich der Mikroelektronik werden an die Reinheit der dort einzusetzenden Diepoxide Qualitätsanforderungen gestellt, wie sie nach dem erfindungsgemäßen Verfahren erfüllt werden.

Das beschriebene Verfahren ist wirtschaftlich, da alle Hilfsmedien rückgeführt werden. Das Verfahren ist besonders umweltfreundlich, da aus dem Oxidationsmittel lediglich Wasser als Abfall entsteht; darüberhinaus fallen nut geringe Mengen an sonstigen Abwässern, Leichtsiedern und Destillationsrückständen an, die unproblematisch und gefahrlos entsorgt werden können.

Erfindungsgemäß sind nur kurze Reaktionszeiten nötig, was die technische Durchführung besonders wirtschaftlich gestaltet.

Es war überraschend und nicht vorhersehbar, daß die Umsetzung des vorgenannten Diolefins mit einer rohen Perpropionsäure, die noch Mineralsäure, Wasser und Wasserstoffperoxid in den vorgenannten Konzentrationen enthält, bei weitestgehender Unterdrückung von Neben-und Folgereaktion durchführbar ist. Weiterhin war nicht vorhersehbar, daß die dabei anfallenden Reaktionsgemische erfindungsgemäß destillativ oder destillativ und desorptiv aufarbeitbar sind, ohne daß sich dadurch der Epoxidgehalt des Produktes merklich verringert.

Beispiel 1 (diskontinuierlich)

Zu 702 g (3 Mol) 2-(Cyclo-3-hexenyl)-spiro(1,3-dioxan-5,3'-cyclohexen), verdünnt mit 300 ml Benzol, wurden unter Rühren und Kühlen auf 30 °C innerhalb 90 Minuten 2575 g (6,3 Mol) Perpropionsäure (22 Gewichtsprozent) in Benzol gegeben. Die Perpropionsäure wurde gemäs DE-PS 25 19 289 hergestellt und enthielt 0,5 Gewichtsprozent $H_2O_2$; 0,93 Gewichtsprozent $H_2O$ und 680 ppm Schwefelsäure. Sie wurde für die Beispiele 1 -4 eingesetzt.

Anschließend rührt man 180 Minuten bei 50 °C nach.

Der Umsatz an Olefin beträgt 99,3 %. Die erhaltene klare, schwach gelbe Lösung wird innerhalb 240 Minuten bei 90 °C und einem Druck von 100 mbar über einen Dünnschichtverdampfer gegeben, wobei gleichzeitig im Gegenstrom ca. 300 g/h Benzoldampf geführt werden. Das so erhaltene Rohepoxid wird nun bei 90 °C/20 mbar über den Dünnschichtverdampfer gegeben; im Gegenstrom wird ein schwacher Stickstoffstrom geführt.

Als Sumpf erhält man 773 g hellgelbes Diepoxid mit einem Epoxidgehalt von 6,73 val/kg und einer Viskosität von 162,3 Pa.s (25 °C).

Beispiel 2 (kontinuierlich)

In den ersten Rührkessel einer Reaktionseinheit, bestehend aus zwei Rührkesseln vom Volumen 1000 ml und 1200 ml, sowie einem als Rohrreaktor ausgebildeten Nachreaktor, der ein Volumen von 790 ml hat, werden stündlich 3,28 Mol Perpropionsäure in Benzol (ca. 22 %ig) und 1,52 Mol 2-(Cyclo-3-hexenyl)-spiro(1,3-dioxan-5,3'-cyclohexen) als Schmelze eingespeist, was einem Molverhältnis Persäure zu Diolefin von 2,16 : 1 entspricht. Die Reaktionstemperatur beträgt im ersten Reaktor 35 °C, im zweiten Reaktor 40 °C und im Nachreaktor 50 °C. Die Umsätze an Olefin betragen nach der Rührkesselkaskade 90,3 %, nach dem Rohrreaktor 98,7 %. Gemäß Aufarbeitungsvariante 2 werden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wird in einem zweiten Verdampfer gleichen Typs und gleicher Fläche bei 90 °C und 100 mbar bei einem Durchsatz von 330 g/h Benzoldampf desorbiert, wobei die Brüden des zweiten Verdampfers im Gegenstrom zum Produktstrom im ersten Verdampfer geführt werden.

Nachfolgend wird das Epoxid in zwei Desorptionseinheiten, bestehend aus jeweils einem Sambay-Verdampfer (Fläche 0,065 $m^2$) bei 100 mbar mit 55 g/h Wasserdampf und bei 20 mbar mit 31 g/h Stickstoff bei Temperaturen von 90 °C behandelt.

Als Produkt fallen stündlich 391,6 g Diepoxid mit folgenden Kennzahlen an:

Viskosität (Pa.s, 25 °C): 158,8

Epoxid-Gehalt (val/kg): 6,83

Farbzahl (Hazen): 60.

Beispiel 3 (kontinuierlich)

In den ersten Rührkessel einer Reaktionseinheit, bestehend aus zwei Rührkesseln vom Volumen jeweils 1500 ml, sowie einem als Rohrreaktor ausgebildetem Nachreaktor, der ein Volumen von 1900 ml hat, werden stündlich 3,39 Mol Perpropionsäure in Benzol (ca. 22 %ig) und 1,54 Mol 2-(Cyclo-3-hexenyl)-spiro(1,3-dioxan-5,3'-cyclohexen) verdünnte mit 150 ml/h Benzol eingespeist, was einem Molverhältnis Persäure zu Diolefin von 2,2 : 1 entspricht. Die Reaktionstemperatur beträgt im ersten Reaktor 41 °C, im zweiten Reaktor 40 °C und im Nachreaktor 50 °C. Die Umsätze an Olefin betragen nach der Rührkesselkaskade 91,7 %,nach dem Rohr reaktor 99,2 %. Gemäß Aufarbeitungsvariante 2 werden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wird in einem zweiten Verdampfer gleichen Typs und gleicher Fläche bei 90 °C und 100 mbar bei einem Durchsatz von 332 g/h Benzoldampf desorbiert und die Brüden entsprechend Beispiel 2 geführt.

Nachfolgend wird das Epoxid in zwei Desorptionseinheiten, bestehend aus jeweils einem Sambay-Verdampfer (Fläche 0,065 $m^2$) bei 100 mbar mit 55 g/h Wasserdampf und bei 20 mbar mit 31 g/h Stickstoff bei Temperaturen von 100 °C behandelt.

Als Produkt fallen stündlich 398,2 Diepoxid mit folgenden Kennzahlen an:

Viskosität (Pa.s, 25 °C): 153,7

Epoxid-Gehalt (val/kg): 6,81

Farbzahl (Hazen): 70.

Beispiel 4 (kontinuierlich)

In den ersten Rührkessel einer Reaktionseinheit, bestehend aus zwei Rührkesseln vom Volumen jeweils 1500 ml sowie einem als Rohrreaktor ausgebildetem Nachreaktor, der ein Volumen von 1900 ml hat, werden stündlich 3,14 Mol Perpropionsäure in Benzol (ca. 22 %ig) und 1,48 Mol 2-(Cyclo-3-hexenyl)-spiro(1,3-dioxan-5,3'-cyclohexen) als Schmelze eingespeist, was einem Molverhältnis Persäure zu Diolefin von 2,12 :1 entspricht. Die Reaktionstemperatur beträgt im ersten Reaktor 40 °C, im zweiten Reaktor 40 °C und im Nachreaktor 50 °C. Die Umsätze an Olefin betragen nach der Rührkesselkaskade 91,5 %, nach dem Rohrreaktor 99,1 %. Gemäß Aufarbeitungsvariante 3 werden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wird in einem zweiten Verdampfer gleichen Typs und gleicher Fläche bei 90 °C und 100 mbar bei einem Durchsatz von 325 g/h Benzoldampf desorbiert, wobei die Brüden aus dem zweiten Verdampfer an dem ersten Verdampfer vorbeigeführt werden. Das so als Sumpf erhaltene Rohepoxid wird nun nach Verdünnen mit 118 g/h Benzol mit einem Mixer-Settler-System mit 1 %iger Natronlauge (180 ml/h) und anschließend in einer Folge von drei Mixer-Settler-Einheiten mit Wasser (jeweils 180 ml/h) gewaschen. Nachfolgend wird das Epoxid in zwei Desorptionseinheiten, bestehend aus jeweils einem Sambay-Verdampfer (Fläche 0,065 $m^2$) bei 80 mbar mit 25 g/h Wasserdampf und bei 80 mbar mit 20 g/h Stickstoff bei Temperaturen von 100 °C behandelt.

Als Produkt fallen stündlich 374,4 g Diepoxid mit folgenden Kennzahlen an:

Viskosität (Pa.s, 25 °C): 149,7

Epoxid-Gehalt (val/kg): 6,95

Farbzahl (Hazen): 40.

**Ansprüche**

1. Verfahren zur Herstellung eines cycloaliphatischen Diepoxids der Formel

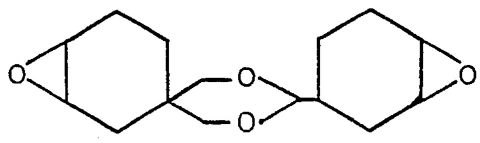

durch Epoxydierung des Diolefins der Formel

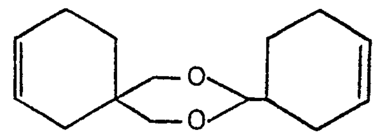

mit einer Percarbonsäure in organischer Lösung, dadurch gekennzeichnet, daß man das Diolefin der obengenannten Formel mit einer Lösung von Perpropionsäure in Benzol im Molverhältnis 1 : 2 bis 1 : 3 (Diolefin zu Perpropionsäure) bei einer Temperatur von 10 bis 100°C, vorzugsweise 20 bis 50 °C, umsetzt.

2. Verfahren nach Anspruch 1, dadurch gekennzeichnet , daß die Perpropionsäurelösung maximal einen Gehalt von 1,5 Gewichtsprozent Wasserstoffperoxid, 1,5 Gewichtsprozent Wasser und ca. 800 ppm Mineralsäure besitzt.

3. Kontinuierliches Verfahren nach den Ansprüchen 1 und 2, dadurch gekennzeichnet, daß man das cycloaliphatische Diolefin mit einer Lösung von Perpropionsäure in Benzol im Molverhältnis 1 : 2 bis 1 : 3 in ein Reaktionssystem einspeist, das aus einer Folge von 1 bis 4 ideal durchmischten Reaktoren und einem Nachreaktor besteht, die Reaktion bei einer Temperatur von 10 bis 100 °C durchführt, wobei man die Verweilzeit so einstellt, daß der Umsatz, bezogen auf eingesetzte olefinische Doppelbindungen, nach dem oder den ideal durchmischten Reaktoren bei mindestens 80 Molprozent und nach dem Nachreaktor bei mindestens 95, bevorzugt>98 Molprozent, liegt, und daß man das aus dem Nachreaktor austretende Gemisch in einer Kombination von Destillations-und Desorptionsschritten von Benzol, Propionsäure, geringen Mengen an Perpropionsäure und von anderen Leichtsiedern befreit.

4. Verfahren nach den Ansprüchen 1 bis 3, dadurch gekennzeichnet, daß man die Destillations- und Desorptionsschritte unter vermindertem Druck von 0,5 bis 600 mbar bei Temperaturen des Heizmediums von 50 bis 150 °C und bei Verweilzeiten von maximal 10 Minuten, bevorzugt maximal 5 Minuten, in den einzelnen Schritten durchführt.

5. Verfahren nach den Ansprüchen 1 bis 4, dadurch gekennzeichnet, daß man die Destillations- und Desorptionsschritte bei 10 bis 300 mbar durchführt.

6. Verfahren nach den Ansprüchen 1 bis 5, dadurch gekennzeichnet, daß man zunächst Benzol und Propionsäure sowie die geringen Mengen Perpropionsäure größtenteils destillativ abtrennt, worauf man die verbliebene Menge Propionsäure im Rohepoxid mit Benzoldampf weiter desorptiv entfernt und entweder hieran direkt anschließend das Benzol und Spuren von Propionsäure desorptiv mit Wasserdampf und/oder Inertgasen austreibt, oder daß man nach der Desoption mit Benzoldampf das rohe Epoxid zunächst mit wäßrigen Alkalien und anschließend mit Wasser wäscht und erst dann die Desorption mit Wasserdampf und/oder Inertgasen anschließt.

7. Verfahren nach den Ansprüchen 1 bis 6, dadurch gekennzeichnet , daß man das aus der Kombination von Destillations-und Desorptionsschritten erhaltene Gemisch aus Benzol, Propionsäure, geringen Mengen Perpropionsäure, sowie gegebenenfalls anderen Leichtsiedern in eine aus zwei oder mehreren Destillationskolonnen bestehende Destillationsanlage führt und in dem ersten Destillationsschritt über Kopf Benzol, gegebenenfalls im Gemisch mit anderen Leichtsiedern, abzieht, das man gegebenenfalls nach destillativer Reinigung in das Herstellungsverfahren der Perpropionsäure wieder zurückführt, und daß man im Sumpf die Gesamtmenge an Perpropionsäure und Propionsäure, sowie Anteile von Benzol in Mengen von 5 bis 35 Gewichtsprozent, bezogen auf die Sumpfmischung, entnimmt und diese Mischung in eine zweite Destillationsstufe führt, in der man die Gesamtmenge des darin enthaltenen Benzols und der Perpropionsäure mit Anteilen an Propionsäure über Kopf abzieht und dabei eine Konzentration von Perpropionsäure in dem Kopfprodukt von mehr als 25 Gewichtsprozent nicht überschreitet, dieses

Kopfprodukt in das Herstellungsverfahren der Perpropionsäure oder in die Umsetzung der Perpropionsäure mit Olefin zurückführt, und überschreitet, dieses Kopfprodukt in das Herstellungsverfahren der Perpropionsäure oder in die Umsetzung der Perpropionsäure mit Olefin zurückführt, und daß man die Perpropionsäure als Sumpfprodukt gegebenenfalls dampfförmig, oberhalb des Sumpfes abzieht und in das Herstellungsverfahren der Perpropionsäure zurückführt.

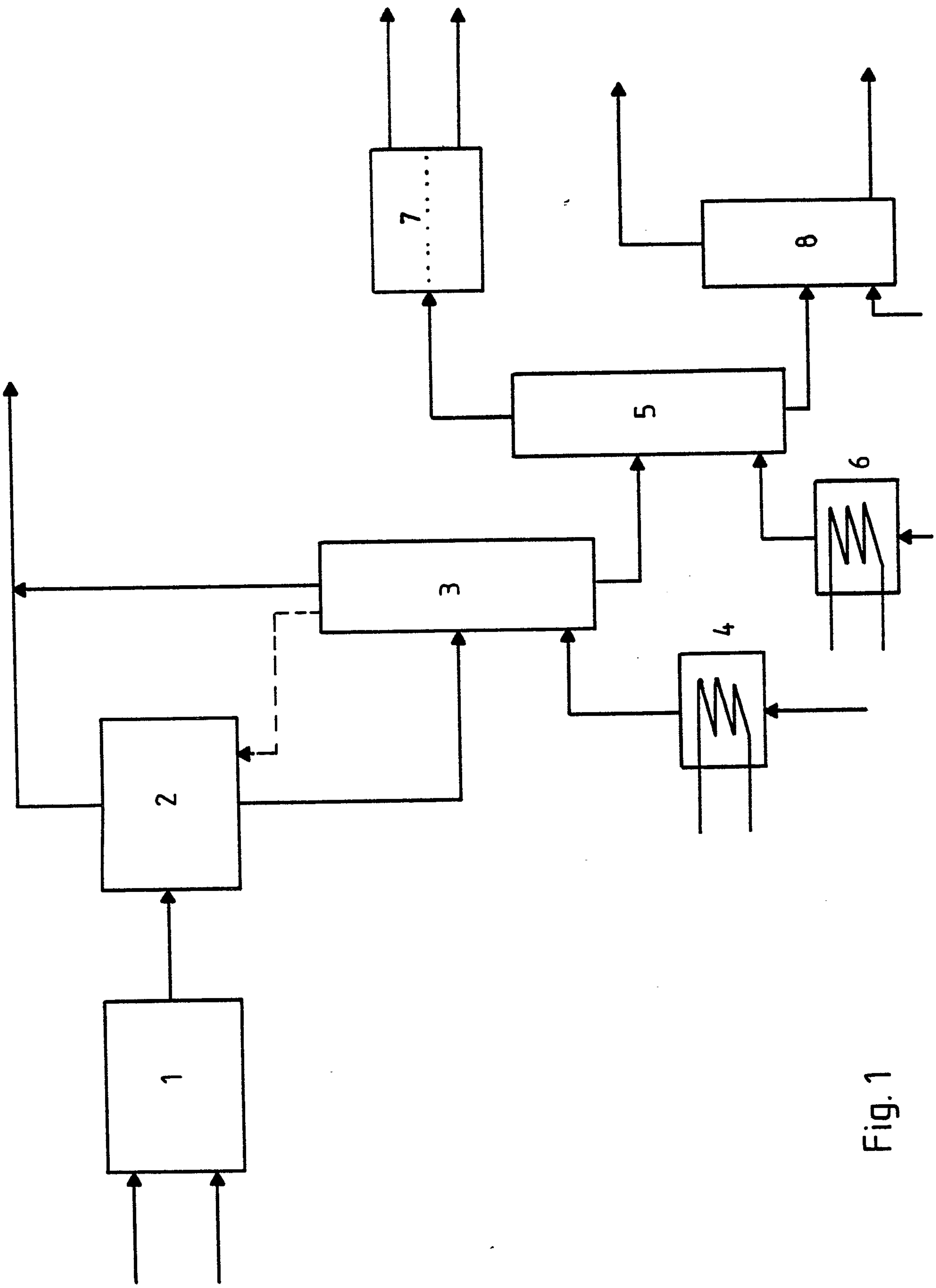


Fig. 1

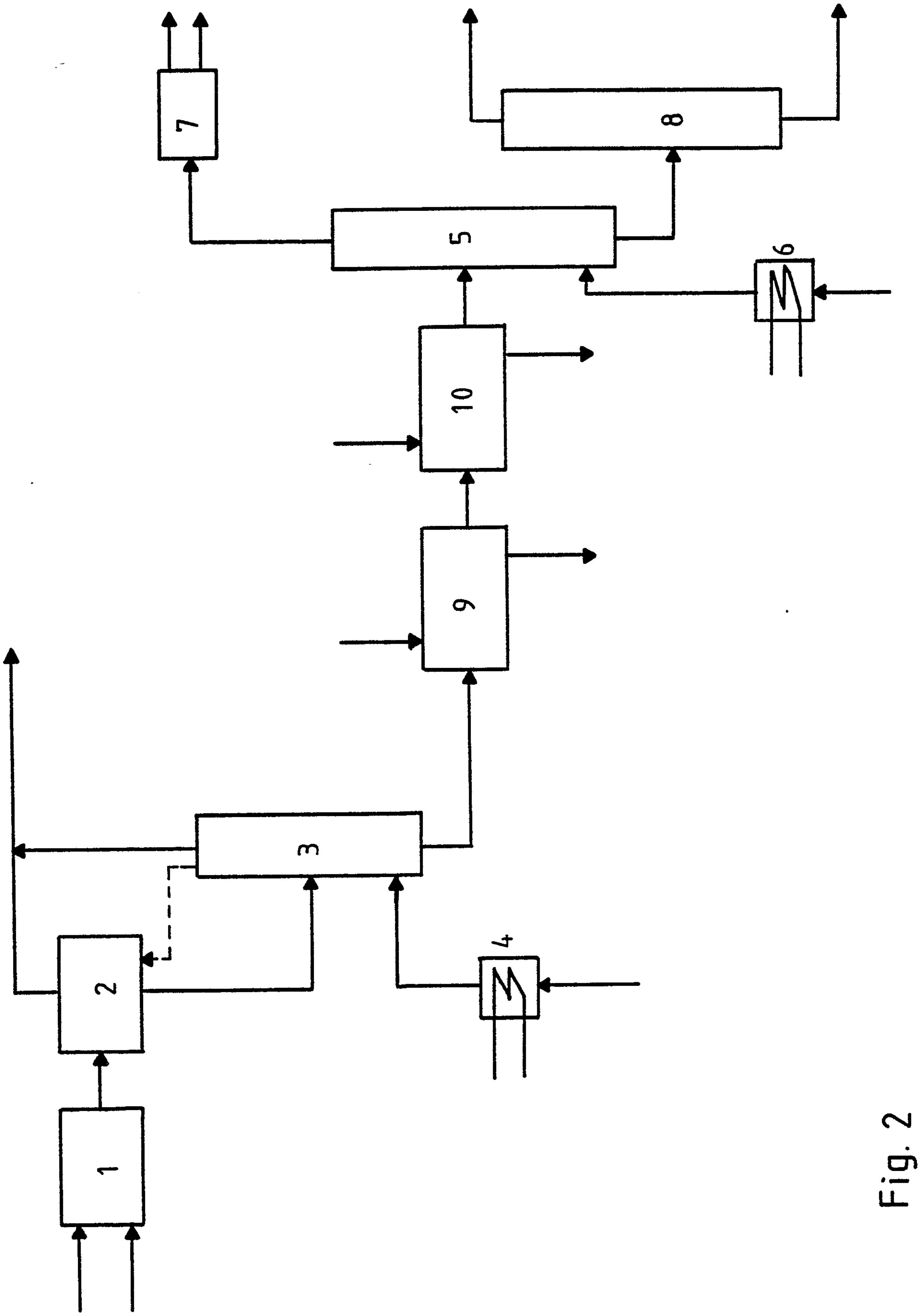


Fig. 2

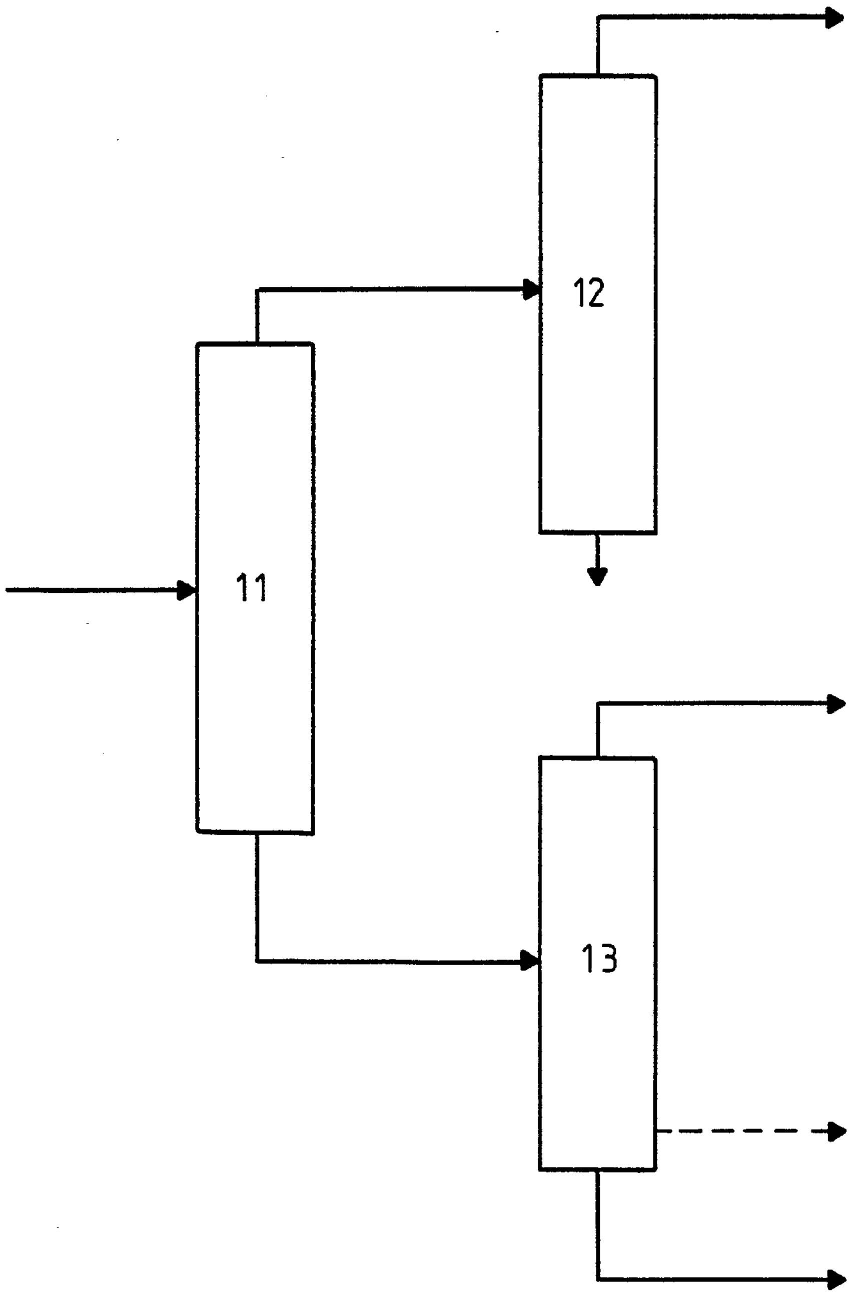


Fig. 3